# EUROPEAN PATENT APPLICATION

(11) **EP 1 930 425 A1**
(43) Date of publication of application: **11.06.2008**
(21) Application number: 06797375.0
(22) Date of filing: 04.09.2006
(51) Int. Cl.: C12N 15/00, C12N 5/10, C12N 15/09

(54) **SEQUENCE CAPABLE OF ENHANCING THE EXPRESSION OF GENE UNDER MODERATELY LOW TEMPERATURE**

(30) Priority: 05.09.2005 JP 2005256085; 08.12.2005 JP 2005355117; 06.06.2006 JP 2006157422
(71) Applicant: Fujita, Jun, Kyoto-shi, Kyoto 6060926 (JP)
(72) Inventor: Fujita, Jun, Kyoto-shi, Kyoto 6060926 (JP)
(74) Representative: Tollervey, Rebecca Marie
(86) International application number: PCT/JP2006/317452
(87) International publication number: WO 2007/029641

(57) **Abstract**

The object is to increase the amount of a protein produced by recombinant DNA method using an eukaryotic cell, particularly a mammalian cell. The object is achieved by using a moderately low temperature transcription control element comprising the nucleotide sequence: X1cccX5X6x7(wherein x1 represents c t or a; x5 represents g, c, a, or t;x6 represents c, t, a or g; and x7 represents, c, a, g, or t) or a nucleotide sequence complementary to the nucleotide sequence or moderately low temperature transcription enhancer having the moderately low temperature transcription control element linked thereto.

## Description

### Technical Field

The present invention relates to mild low temperature transcription control elements (oligonucleotides) enhancing the transcription of a gene at mild low temperatures, and an enhancer comprising the mild low temperature transcription control elements. The present invention also relates to a method for assaying and purifying mild low temperature transcription control factors (proteins) using mild low temperature transcription control elements.

### Background Art

Various pharmaceuticals and assay reagents such as monoclonal antibodies, growth factors and clotting factors have been produced by biotechnology.

Generally, at a low temperature of 10-15°C, correct folding of heterologous recombinant protein is promoted and degradation of the protein by proteases is inhibited (Baneyx, F., In vitro folding of recombinant proteins in Escherichia coli. In Manual of industrial microbiology and biotechnology, 2nd ed., (Demain, A. L. Davis,J.E.,Altas, R.M., et al., Eds.) ASM Press, Washingtonn D. C., pp.551-1999). Accordingly, when recombinant proteins are produced by bacteria, cultivation at low temperatures below 20°C is effected, instead of at 37°C. In this case, since protein synthesis is generally lowered at low temperature, possible low yield of a desired protein may occur. This problem is solved by using the promoter of cold shock protein CspA gene and 5' untranslated region (UTR) of CspA mRNA in an expression vector of a desired protein (Baneyx, F. and Mujacic, M., Cold-inducible promoters for heterologous protein expression, Methods in Molecular Biology, 205, 1-18, 2001) .

However, posttranslational modification in bacteria is significantly different from that in mammalian cells. Especially for pharmaceuticals, glycosylation state is important for therapeutic effects. Therefore, mammalian cells, particularly Chinese hamster cell lines, are used for producing protein pharmaceuticals. It is necessary to increase protein yield in order to reduce production cost with the cell cultivation. When usual cultivation at 37°C is effected, there is a disadvantage that, since cells die after rapid growth, the term during which cells survive and produce the desired protein is short. Therefore, an attempt to restrain cell growth and have cells survive for a longer time period is effected by the addition of cell growth inhibitors, the expression of cell-cycle inhibitory gene p27, the cultivation at mild low temperatures (30-32°C), etc. (see Schatz SM et al., Biotechnol. Bioeng, 2003, Nov. 433-438).

Among them, the cultivation at mild low temperature has the characteristics as follows:
1) Cells survive and produce recombinant proteins for a longer time period than the cultivation at 37°C;
2) The activity of proteolytic enzyme is lowered to reduce the degradation of desired proteins;
3) Glycosylation and isoprotein patterns are similar to those cultivated at 37°C, and sialylation is rather better than that cultivated at 37°C;
4) A possible improvement in correct folding of desired proteins as in bacteria.
   (Kaufmann H, Mazur X, Marone R, Bailey JE, Fussengger M., Biotech Bioeng., 2001 Mar. 20, 72(6):592-602; Yoon SK, Song JY, Lee GM., Biotech Bioeng., 2003 May 5, 82(3):289-98).

In regard to crucial yield of recombinant protein, since cell survive terms is extended, total yield is often increased. However, the specific productivity of protein per cell per unit time varies for unknown reasons, possibly depending on kinds of cell, kinds of the desired protein, and others. There are many cases in which the specific productivity is rather lower than that at 37°C, which is problematic (Kaufmann H, Mazur X, Marone R, Bailey JE, Fussenegger M., Biotech Bioeng., 2001 Mar. 20, 72(6):592-602; Yoon SK, Song JY, Lee GM., Biotech Bioeng., 2003 May. 5, 82(3):289-98). Since gene expression control mechanism is different from that in bacteria, cold shock protein CspA promoter and 5' untranslated region (UTR) of CspA mRNA in bacteria cannot be used in mammalian cells.

On the other hand, a yeast which is the simplest eukaryote has been used as a model of a human in bioscientific study by the following reasons:
a yeast has all fundamental functions as an eukaryote although the genome size thereof is as small as 13 million base pairs;
more than 30% of yeast genes has introns;
amino acid sequence of yeast gene is highly conserved with the known human homologous gene to normally express human gene in yeast; and
functional analysis is easy (Molecular Biology of the Cell, 4th edition, Alberts B, Johnson A, Lewis J, Raff M, Roberts, K, Walter P, published by Garland Science, New York, 2002).

Thus, fungi including yeasts as well as cells from eukaryotes closer to humans than fungi, for example, plants, roundworms, insects, fishes, amphibians, and birds are considered to be able to carry out post-translational modification of proteins like mammalian cells by appropriate supplementation of genes as necessary and produce proteins at low culture temperatures. In addition, when genetic engineering technique is used, it would be possible to obtain eukaryote individuals containing eukaryote cells subjected to gene modification and to produce protein.
Non-patent literature 1: Kaufmann H, Mazur X, Marone R, Bailey JE, Fussenegger M., Biotech Bioeng., 2001 Mar. 20, 72(6):592-602;
Non-patent literature 2: Yoon SK, Song JY, Lee GM., Biotech Bioeng., 2003 May. 5, 82(3):289-98;
Non-patent literature 3: Fujita J., J. Mol. Microbiol. Biotechnol., 1999 Nov 1(2): 243-55
Non-patent literature 4: Molecular Biology of the Cell, 4th edition, Alberts B, Johnson A, Lewis J, Raff M, Roberts K, Walter P, published by Galrand Science, New York, 2002

### Disclosure of Invention

### Problem to Be Solved

It is an object of the present invention to provide a method for increasing the amount of protein produced per cell per unit time and total yield at mild low temperatures.

It is also an object of the present invention to provide a method for assaying and purifying mild low temperature transcription control factors (proteins).

### Means for Solving Problem

As disclosed in the pending international patent application (PCT/JP2005/003385), the present inventor has found a gene expression control sequence which acts as an enhancer at mild low temperatures (referred to as mild low temperature transcription control element). The example of the sequence is a nucleotide sequence of:
tccccgcc
or a sequence complementary to the sequence.

The present inventor has further carried forward the study on mild low temperature transcription control elements. As a result, it has been found that a nucleotide sequence of:
X1cccX5X6X7
wherein X1 represents c, t, or a; X5 represents g, c, a, or t; X6 represents c, t, a, or g; and X7 represents c, a, g or t,
specifically enhances transcription of gene at mild low temperature.

That is, the present invention relates to mild low temperature transcription control elements consisting of a nucleotide sequence of:
X1cccX5X6X7
wherein X1 represents c, t, or a; X5 represents g, c, a, or t; X6 represents c, t, a, or g; and X7 represents c, a, g or t,
or a nucleotide sequence complementary to this sequence.

The term "mild low temperature transcription control element" refers to a nucleotide sequence capable of accelerating the transcription of a gene specifically at mild low temperatures.

The term "mild low temperature" herein refers to a temperature of 15-36°C, preferably a temperature of 30-34°C and most preferably a temperature of 32°C. The mild low temperature transcription control element functions as a mild low temperature specific enhancer for transcription of genes.

The mild low temperature transcription control element of the present invention can exhibit its function when it is used alone. However, it is preferable to use it in connection of 2-350, preferably 2-100 of the same or different elements. When the elements are used in connection, the same or different elements may be directly connected, or may be connected via any number and any kind of nucleotides. The connected mild low temperature transcription control elements are referred to as "mild low temperature transcription enhancer" or only "enhancer" in the present specification. The mild low temperature transcription enhancer of the present invention excludes nucleotide sequence which contains only the nucleotide sequence of ccccgcc, ccccgtc, ccccgct or ccccgaa which follow 3' to t; the nucleotide sequence of ccccgcc which follow 3' to a; the nucleotide sequence of ccccgcc which follow 3' to a; the nucleotide sequence of ggcgggg or ggggggg which precede a at 3'terminus as mild low temperature transcription control elements.

The mild low temperature transcription control element and the mild low temperature transcription enhancer of the present invention have no relation to the distance and the direction from a promoter.

The term "mild low temperature transcription control factor (protein)" refers to a protein which is activated at mild low temperature and binds to mild low temperature transcription control elements or enhancer to control, in many cases, accelerate the transcription of gene present at the neighborhood of the element.

The present invention relates to a vector comprising the mild low temperature transcription control element or the mild low temperature transcription enhancer described above.

The present invention relates to eukaryote cells transformed by the vector.

The present invention relates to eukaryotes transformed by the vector. As shown in Example hereinafter, mild low temperature transcription enhancer can be stably integrated into cell chromosome.

The present invention relates to a method for assaying mild low temperature transcription control factors (proteins) and the regulatory substances thereof.

The method comprises:
incubating double-stranded DNA probe containing mild low temperature transcription control elements with protein solution to be assayed;
measuring the amount of mild low temperature transcription control factors specifically bound to mild low temperature transcription control elements by detecting the DNA probe bound to the proteins.

The activity of a regulatory substance is assayed by quantifying the change of the amount of mild low temperature transcription control factors bound to the DNA probe.

The present invention relates to a method for purifying mild low temperature transcription control factors. The method comprises:
incubating double-stranded DNA probe containing mild low temperature transcription control elements with protein solution;
obtaining mild low temperature transcription control factors (proteins) specifically bound to mild low temperature transcription control elements; and
isolating mild low temperature transcription control factors.

### Effect of the Present Invention

The present invention can accelerates the transcription of a gene at mild low temperatures. According to the present invention, mild low temperature transcription control factors (proteins) and regulatory substances thereof can be assaying or purified.

### Brief Description of Drawings

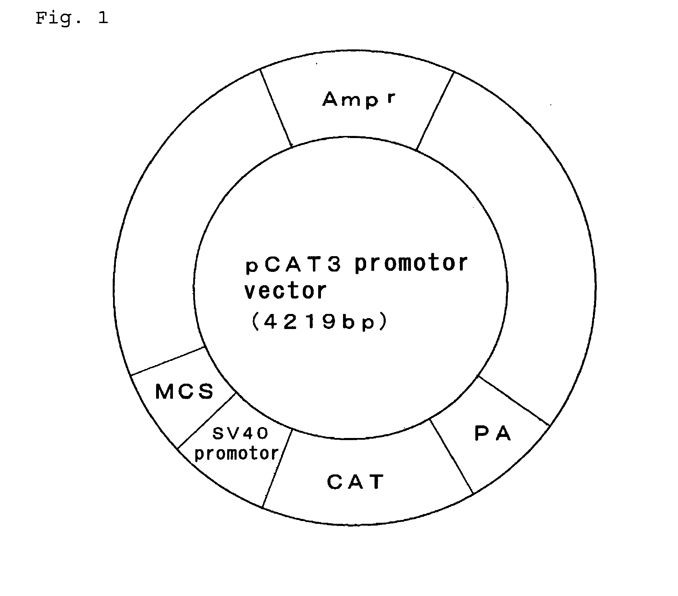

Fig. 1 is a schematic view representing pCAT3 promoter vector. MCS represents a multi-cloning site. SV 40 promoter represents a promoter derived from SV40 virus. CAT represents a coding sequence of chloramphenicol acetyltransferase. PA represents a polyadenylation signal derived from SV40 virus. Amp^{r} represents ampicillin resistance gene.

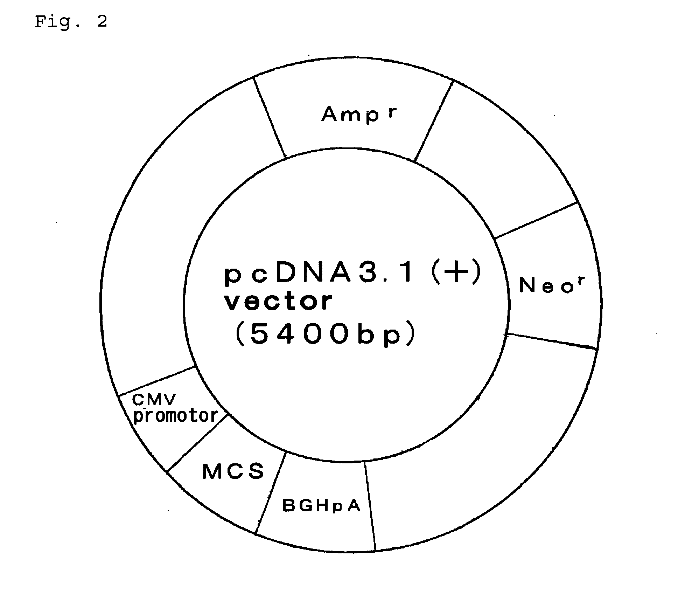

Fig. 2 is a schematic view representing pcDNA 3.1(+) vector (Invitrogen). MCS represents a multi-cloning site. Firefly luciferase encoding sequence is inserted between EcoRV and XbaI of the multi-cloning site. A mild low temperature transcription control enhancer is integrated 5' to CMV promoter. Neo^{r} represents G418 resistance gene.

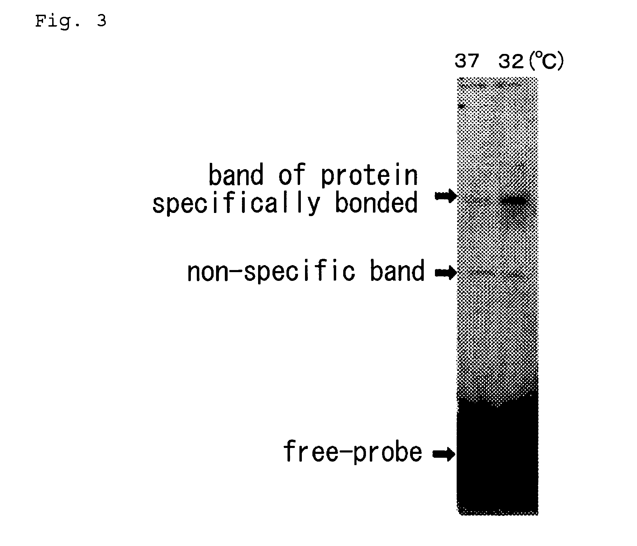

Fig.3 represents a result of gel shift assay. Proteins extracted from mouse Balb/3T3 cells cultured at 37°C (1 lane) or 32°C (2 lane) for 8 hours were analyzed by gel shift assay using 32-P-ATP-labeled oligonucleotides 5'-ttccccgccgacggatccag-3' as a probe to detect mild low temperature transcription control factors bound to the mild low temperature transcription control element or enhancer.

### Best Mode for Carrying Out the Invention

Examples of the mild low temperature transcription control element of the present invention include, but is not limited to:
ccccgcc, ccccgca, ccccgcg, ccccgct, ccccgtc, ccccgta, ccccgtg, ccccgtt, ccccgac, ccccgaa, ccccgag, ccccgat, ccccggc, ccccgga, ccccggg, ccccggt, ccccccc, cccccca, ccccccg, cccccct, ccccctc, cccccta, ccccctg, ccccctt, cccccac, cccccaa, cccccag, cccccat, cccccgc, cccccga, cccccgg, cccccgt, ccccacc, ccccaca, ccccacg, ccccact, ccccatc, ccccata, ccccatg, ccccatt, ccccaac, ccccaaa, ccccaag, ccccaat, ccccagc, ccccaga, ccccagg, ccccagt, cccctac, cccctcc, tcccgcc, tcccgca, tcccgcg, tcccgct, tcccgtc, tcccgta, tcccgtg, tcccgtt, tcccgac, tcccgaa, tcccgag, tcccgat, tcccggc, tcccgga, tcccggg, tcccccc, tccccca, tcccccg, tccccct, tcccctc, tccccta, tcccctg, tcccctt, tccccac, tccccaa, tccccag, tccccat, tccccgc, tcccacc, tcccaca, tcccacg, tcccact, tcccatc, tcccata, tcccatg, tcccatt, tcccaac, tcccaaa, tcccaag, tcccaat, tcccagc, tcccaga, tccctcc, acccgcc, acccgca, acccgcg, acccgct, acccgtc, acccgta, acccgtg, acccgtt, acccgac, acccgaa, acccgag, acccgat, acccggc, acccgga, acccggg, acccggt, acccccc, accccca, acccccg, accccct, acccctc, accccta, acccctg, acccctt, accccac, accccaa, accccga, acccacc, acccaca, acccacg, acccact, acccatc, acccata, acccatg, acccatt, acccaac, acccaaa, acccaag, acccaat, gcccccc, ggcgggg, agcgggg, gacgggg, ttcgggg, ggggggg, ggcggga, tgcggga, gacggga, tggggga, and ggcgggt.

### (i) Preparation of Mild Low Temperature Transcription Control Elements

The mild low temperature transcription control element, and the mild low temperature transcription enhancer of the present invention can be prepared, for example, via a solid synthesis method by phosphoroamidide method or via a solid synthesis method by hydrogen phosphonate method, using four kinds of deoxyribonucleotide with protective groups (see, for example, Oligonucleotide Synthesis: A Practical Approach, IRL Press, 35-81,83-115 (1984); J. Org. Chem., 49, 2139 (1984); Tetrahedron Lett., 27, 469 (1986); Tetrahedron Lett.,22, 1859-1862; Tetrahedron Lett., 21, 719-722; J. Am. Chem. Soc., 103, 3185-3191 (1981). Nowadays they can be easily synthesized with commercially available DNA synthesizers (for example, manufactured by Applied Biosystem) .

### (ii) Promoter to Be Used

Prokaryote promoters, eukaryote promoters and virus promoters can be used as promoters. Mammalian promoters and virus promoters are preferred. Examples of preferred promoters include mouse thymidine kinase promoter (TK), cytomegalovirus (CMV) promoter and SV40 promoter.

### (iii) Coding Sequence

Genes to be expressed are not limited, and the nucleotide sequence encoding any protein can be used. Noncoding RNAs can be expressed as well.

### (iv) Vector to Be Used

The mild low temperature transcription control element or the mild low temperature transcription enhancer of the present invention, a promoter and the coding sequence encoding a protein to be expressed are linked together and inserted into a vector. The expression vectors for prokaryote cells and eukaryote cells can be used. Examples of preferred vector include, but not limited to, pCMV (Invitrogen), pCAT3 promoter vector (Promega) and pSV2-neo vector.

### (v) Host Cell to Be Used

Host cells are transfected with the vector. It is preferred to use eukaryote cells, in particular mammalian cells as host cells. Examples of preferred mammalian cell are human cell lines such as 293, 293T, U-2OS, Huh-7, HeLa ; monkey cell lines such as COS-7 and Vero; mouse cell lines such as NIH/3T3, Balb/3T3, B-6, Hepal-6 and Chinese hamster cell lines such as CHO.

The methods for the transfection include calcium phosphate method, lipofection method, retrovirus method and electroporation method. For example, in the calcium phosphate method, calcium chloride solution containing DNA (vector) is added dropwise to phosphoric acid solution with stirring. Fine precipitation containing DNA-calcium phosphate is formed. When the solution is added to host cell, the precipitation of DNA-calcium phosphate enters into host cells via phagocytosis so that DNA is transfected into cells.

### (vi) Cultivation Condition

The host cell is cultivated at a mild low temperature. It is unnecessary to use special medium as culture medium. Usual medium for mammalian cell cultivation can be used. Typical example of the medium is Dulbecco's modified Eagle's medium (D-MEM) supplemented with 10% fetal calf serum (FCS) (Invitrogen).

The present invention relates to a method for assaying mild low temperature transcription control factors.
The method comprises:
incubating double-stranded DNA probe containing mild low temperature transcription control elements with protein solution to be assayed;
measuring the amount of mild low temperature transcription control factors by detecting the double-stranded DNA probe bound to them.

The activity of regulatory substance is assayed by quantifying the change of the amount of mild low temperature transcription control factors bound to the DNA probe.

First, DNA oligonucleotide containing mild low temperature transcription control elements and complementary strand are prepared according to conventional method. The terminus of oligonucleotide prepared is labeled, for example, with biotin, and the labeled oligonucleotides are annealed to be double stranded. Alternatively, it is also possible to label oligonucleotides after they are made double stranded; or to use double-stranded oligonucleotides without labeling to be detected at the last step. Commercially available gel shift assay (EMSA: electrophoretic mobility shift assay) kits (PIERCE, Molecular Probes) can be utilized. Then, double-stranded oligonucleotides are mixed with mild low temperature transcription control factors (proteins), or occasionally protein solution which is considered to contain the controlling substances thereof, for example, nuclear extracts to react double-stranded oligonucleotides with mild low temperature transcription control factors (proteins). Then oligonucleotides, proteins and oligonucleotides bound to proteins are separated by using gel elctrophoresis, for example, polyacrylamide gel elctrophoresis. Free oligonucleotides are positioned at the lower part since they move fast in the gel, whereas oligonucleotides bound to proteins appear at the upper part since they move slowly. The mild low temperature transcription control factors are detected as proteins specifically bound to the oligonucleotides. For example, when oligonucleotide is labeled with biotin, polyacrylamide gel is blotted to a nylon membrane after electrophoresis, and then proteins and oligonucleotides transferred are cross-linked to the membrane using ultraviolet. The biotin label immobilized on the membrane is specifically bound to peroxidase-labeled streptoavidin, and emits light by the reaction with chemiluminescent substrate to be detected after exposure to X-ray film.

When the above kit of Molecular Probes' is used, oligonucleotides bound to proteins can be detected by incubating non-labeled oligonucleotides with proteins, subjecting them to elecrophoresis, and staining the gel with dye in situ. From the amount of oligonucleotides specifically bound to protein, the amount of mild low temperature transcription control factors is measured. It is also possible to assay the activity of a regulatory substance by quantifying its effect on the amount of low temperature transcription control factors.

The present invention relates to a method for purifying mild low temperature transcription control factors.
The method comprises:
incubating double-stranded DNA probe containing mild low temperature transcription control elements with protein solution;
obtaining mild low temperature transcription control factors (proteins) specifically bound to mild low temperature transcription control elements; and
isolating mild low temperature transcription control factors.

First, DNA oligonucleotide containing mild low temperature transcription control elements and complementary chain thereof are prepared according to conventional method. Then, the oligonucleotides prepared are immobilized, for example, by linking to carriers. For example, oligonucleotide is biotinylated and annealed to complementary chain to become double stranded, which is linked to avidinylated magnetic beads. Then, immobilized double-stranded oligonucleotides and a protein solution possibly containing mild low temperature transcription control factors, for example, size-fractionated nuclear protein extracts are mixed to allow contact of double-stranded oligonucleotides with mild low temperature transcription control factors. Then, double-stranded oligonucleotides with bound mild low temperature transcription control factors are recovered, for example, with magnet when magnetic beads are used as carriers. After extensive washing of the beads, specifically bound proteins i.e., mild low temperature transcription control factors (proteins), are eluted and purified. If necessary, mild low temperature transcription control factor is further purified using gel elctrophoresis and column chromatography. The amino acid sequence and the mild low temperature transcription control factor gene can be identified from the purified protein.

The present invention is further explained in detail referring to example. However, the present invention is not limited to the examples.

### Example 1

An oligonucleotide prepared by connecting three of eight bases containing tccccgcc in series (tccccgcctccccgcctccccgcc) (SEQ ID NO:1), or an oligonucleotide prepared by connecting three of eight bases in series in which t of 5' terminus of tccccgcc is changed to other base was inserted into the multicloning site of pCAT promoter vector (Promega). This vector had the promoter of SV40 and chloramphenicol acetyltransferase (CAT) gene as a reporter protein. pCAT promoter vector which had no insert was used as a negative control.

Each 2.5-5.0 µg /35 mm dish of these plasmids was co-transfected into human cell line 293 with 0.5-1.0 µg / 35 mm dish of CDM8-LacZ (which is a galactosidase-expressing plasmid and was used to amend the each transfection efficiency, ATCC)

The transfected cells were divided into two parts and one part was cultured at 32°C and the other part was cultured at 37°C. The medium used was D-MEM supplemented with 10% FCS.

After 36 hours, cells were recovered by centrifugation and total cell lysates were prepared. The amount of chloramphenicol acetyltransferase (CAT) protein produced at respective temperature was determined using CAT ELIZA kit (Roche Diagnostics) and the β-galactosidase (LacZ) activity was determined using β-galactosidase assay kit (Stratagene).

The data at 36 hours are shown below. Transcriptional activity was determined by dividing the amount of CAT protein by β-galactosidase activity. The ratio of transcriptional activity at 32°C/ transcriptional activity at 37°C obtained with pCAT promoter vector with no insert was supposed to be 1. The mild low temperature trancription enhancing (enhancer) activity of each variant is shown in Table 1.

**Table 1**

| Mild Low Temperature Transcription Enhancing (Enhancer) Activity of Variant in Which the First One Base is Mutated | | |
|---|---|---|
| Element | Base Sequence | Enhancer Activity |
| Wild type 0t | tccccgcc | 5.8 |
| Variant 0c | Cccccgcc | 4.5 |
| Variant Oa | Accccgcc | 5.5 |
| Variant Og | Gccccgcc | 5.5 |
| control | | 1.0 |

Variants in which the first base was changed to C, A or G exhibited almost same mild low temperature transcription enhancing activity as wild type. This fact shows that mild low temperature transcription control element is functional enough when it is ccccgcc consisting of 7 nucleotides.

When ttccccgccgttccccgccgttccccgccg(SEQ ID NO:2) was used in place of the mild low temperature transcription enhancer tccccgcctccccgcctccccgcc (SEQ ID NO:1), similar results were obtained.

### Example 2

It was found that even if the first base in eight bases is any of T, C, A and G, the change has no effect on the mild low temperature transcription enhancing activity. Accordingly an oligonucleotide was prepared by connecting three of oligonucleotide containing eight bases in series in which T, C, A, G was attached to the 5' side of base sequence of tcccgcc, acccgcc, gcccgcc, ccccgct, respectively, and was inserted into the multicloning site of pCAT promoter vector (Promega), and mild low temperature transcription enhancing activity was measured to obtain the result shown in Table 2. Transcriptional activity was determined by dividing the amount of CAT protein by β-galactosidase activity. The ratio of transcriptional activity at 32°C/ transcriptional activity at 37°C obtained with pCAT promoter vector with no insert was assumed to be 1. In addition, as a positive control, three of wild type tccccgcc were connected in series and the ratio of 32°C/37°C was amended to be 5 to give mild low temperature transcription enhancing activity of each variant.

**Table 2**

| Effect of First One Base on Mild Low Temperature transcription Enhancing (Enhancer) Activity | | |
|---|---|---|
| Element | base sequence | enhancer activity |
| Wild type 0t | tccccgcc | 5.0 |
| Variant 0t1t | tTcccgcc | 5.1 |
| Variant 0c1t | CTcccgcc | 4.6 |
| Variant 0a1t | ATcccgcc | 4.8 |
| Variant 0g1t | GTcccgcc | 4.9 |
| Variant 0t1a | tAcccgcc | 3.1 |
| Variant 0c1a | CAcccgcc | 3.0 |
| Variant 0a1a | AAcccgcc | 2.9 |
| Variant 0g1a | GAcccgcc | 2.8 |
| Variant 0t1g | tGcccgcc | 1.1 |
| Variant 0c1g | CGcccgcc | 1.0 |
| Variant 0a1g | AGcccgcc | 1.1 |
| Variant 0g1g | GGcccgcc | 1.2 |
| Variant Ot7t | tccccgcT | 4.8 |
| Variant Oc7t | CccccgcT | 4.8 |
| Variant Oa7t | AccccgcT | 5.2 |
| Variant Og7t | GccccgcT | 5.0 |
| | | |
| negative control | | 1.0 |

It was confirmed again that a difference, T, C, A or G, in the first base of mild low temperature transcription control element 8 bases, for example, tccccgcc, does not affect the activity. Accordingly, mild low temperature transcription enhancer could be formed, based on 7 bases, for example, ccccgcc instead of 8 bases. When ccccgccccccgccccccgcc (SEQ ID NO: 4) was used in place of the above mild low temperature transcription enhancer tccccgcctccccgcctccccgcc (SEQ ID NO: 1), similar results were obtained.

### Example 3

One base c of the first, second, third, or fourth from the 5' terminus was changed to t, a or g, tt was added to the 5' side, and g or t was added to the 3' side of the seven base sequence ccccgcc to make it ten bases. Three of the ten bases were connected in series and inserted into multicloning site of pCAT promoter vector (Promega). Then, procedure similar to example 2 was carried out to give the result represented in Table 3.

**Table 3**

| Effect of One Base Mutation of the First, Second, Third or Fourth Base on Mild Low Temperature Transcription Enhancing (Enhancer) Activity | | |
|---|---|---|
| Element | base sequence | Enhancer Activity |
| Wild Type | ccccgcc | 5.0 |
| Variant 1a | Acccgcc | 3.0 |
| Variant 1g | Gcccgcc | 1.1 |
| Variant 1t | Tcccgcc | 5.1 |
| Variant 2a | cAccgcc | 1.3 |
| Variant 2g | cGccgcc | 1.0 |
| Variant 2t | cTccgcc | 1.1 |
| Variant 3a | ccAcgcc | 1.0 |
| Variant 3g | ccGcgcc | 1.3 |
| Variant 3t | ccTcgcc | 1.2 |
| Variant 4a | cccAgcc | 1.2 |
| Variant 4g | cccGgcc | 1.2 |
| Variant 4t | cccTgcc | 1.1 |
| | | |
| negative control | | 1.0 |

The variant in which the first base was changed to T had the similar mild low temperature transcription enhancing activity to wild type. The variant in which the first base was changed to A had a somewhat lower mild low temperature transcription enhancing activity compared to wild type, whereas the variant in which the first base was changed to G had a low activity. The variant in which the second, third or fourth base was changed to A, G, or T had a low activity.

### Example 4

Mild low temperature transcription enhancing activity was examined when one more base was mutated in addition to change of the first base C of ccccgcc to T. That is, oligonucleotide containing three variants in series respectively:
a variant in which the fifth base was changed to A, C, or T
in addition to variation of the first base to T, respectively;
a variant in which the sixth base was changed to A, G, or T
in addition to variation of the first base to T, respectively;
a variant in which the seventh base was changed to A, G, or T in addition to variation of the first base to T respectively;
was incorporated into the multi-cloning site of pCAT3 promoter vector (Promega) as in Example 3 to give the following result shown in Table 4.

**Table 4**

| Mild Low Temperature Transcription Enhancing (Enhancer) Activity of Two-Base Variants. | | |
|---|---|---|
| Element | Base Sequence | Enhancer Activity |
| wild type | ccccgcc | 5.0 |
| Variant 1t5a | TcccAcc | 2.1 |
| Variant 1t5c | TcccCcc | 2.3 |
| Variant 1t5t | TcccTcc | 1.6 |
| Variant 1t6a | TcccgAc | 2.1 |
| Variant 1t6g | TcccgGc | 2.0 |
| Variant 1t6t | TcccgTc | 3.1 |
| Variant 1t7a | TcccgcA | 2.8 |
| Variant 1t7g | TcccgcG | 2.6 |
| Variant 1t7t | TcccgcT | 2.6 |
| | | |
| negative control | | 1.0 |

When one base variation existed in addition to variation of the first base to T, mild low temperature transcription enhancing activities of variant of the fifth base to C, A or T; variant of the sixth base to T, A or G; variant of the seventh base to A, G or T were lowered compared with that of wild type, but had significant activities.

### Example 5

From the above results, a nucleotide sequence of:
X1cccX5X6X7
wherein X1 represents c, t, or a; X5 represents g, c, a, or t; X6 represents c, t, a, or g, X7 represents c, a, g, or t,
was mild low temperature transcription control element, an oligonucleotide sequence in which they were connected was enhancer, and nucleotide sequence complementary to these was considered to have mild low temperature transcription enhancing activity. Accordingly, three molecules of single oligonucleotide containing a nucleotide sequence which conformed or did not conform to the mild low temperature transcription control element sequence were connected in series, and were incorporated into pCAT3 promoter vector (promega) as in Example 3 to obtain the following result shown in Table 5.

**Table 5**

| Mild Low Temperature Transcription Enhancing (Enhancer) Activity of Various Variants | | | | | | | |
|---|---|---|---|---|---|---|---|
| **Mild Law Temperature Transcription Enhancing (Enhancer) Activity of Various Variant** | | | | | | | |
| base sequence | activity | base sequence | activity | base sequence | activity | base sequence | activity |
| ccccgcc | 5.0 | ccccgcA | 3.5 | ccccgcG | 2.7 | ccccgcT | 5.1 |
| ccccgTc | 4.9 | ccccgTA | 3.6 | ccccgTG | 2.8 | ccccgTT | 4.0 |
| ccccgAc | 4.5 | ccccgAA | 4.8 | ccccgAG | 2.2 | ccccgAT | 3.0 |
| ccccgGc | 2.0 | ccccgGA | 2.1 | ccccgGG | 2.0 | ccccgGT | 2.1 |
| ccccCcc | 2.9 | ccccCcA | 2.3 | ccccCcG | 2.9 | ccccCcT | 2.8 |
| ccccCTc | 2.3 | ccccCTA | 2.0 | ccccCTG | 2.0 | ccccCTT | 2.2 |
| ccccCAc | 2.2 | ccccCAA | 2.0 | ccccCAG | 2.0 | ccccCAT | 2.1 |
| ccccCGc | 3.1 | ccccCGA | 2.5 | ccccCGG | 2.4 | ccccCGT | 2.8 |
| ccccAcc | 2.9 | ccccAcA | 1.9 | ccccAcG | 1.9 | ccccAcT | 2.2 |
| ccccATc | 2.1 | ccccATA | 2.0 | ccccATG | 1.9 | ccccATT | 2.1 |
| ccccAAc | 2.1 | ccccAAA | 2.1 | ccccAAG | 2.1 | ccccAAT | 2.0 |
| ccccAGc | 2.1 | ccccAGA | 2.1 | ccccAGG | 2.0 | ccccAGT | 2.0 |
| ccccTAc | 1.5 | ccccTAA | 1.3 | ccccTGc | 1.1 | ccccTcc | 1.5 |
| Tcccgcc | 5.1 | TcccgcA | 2.8 | TcccgcG | 2.6 | TcccgcT | 2.6 |
| TcccgTc | 3.1 | TcccgTA | 2.9 | TcccgTG | 2.5 | TcccgTT | 2.4 |
| TcccgAc | 2.1 | TcccgAA | 2.5 | TcccgAG | 2.0 | TcccgAT | 2.9 |
| TcccgGc | 2.0 | TcccgGA | 1.4 | TcccgGG | 1.5 | TcccCcc | 2.5 |
| TcccCcA | 2.5 | TcccCcG | 2.9 | TcccCcT | 2.3 | TcccCTc | 2.2 |
| TcccCTA | 2.2 | TcccCTG | 1.9 | TcccCTT | 2.0 | TcccCAc | 1.9 |
| TcccCAA | 2.0 | TcccCAG | 2.0 | TcccCAT | 2.1 | TcccCGc | 1.9 |
| TcccCGA | 1.3 | TcccAcc | 2.1 | TcccAcA | 1.9 | TcccAcG | 2.0 |
| TcccAcT | 2.0 | TcccATc | 2.0 | TcccATA | 1.9 | TcccATG | 1.9 |
| TcccATT | 2.0 | TcccAAc | 1.9 | TcccAAA | 2.0 | TcccAAG | 2.0 |
| TcccAAT | 2.1 | TcccAGc | 1.9 | TcccAGA | 1.4 | TcccAGG | 1.2 |
| TcccTcc | 1.6 | TcccTcA | 1.2 | TcccTcG | 1.1 | TcccTcT | 1.1 |
| TcccTTc | 1.2 | TcccTAc | 1.2 | TcccTGc | 1.0 | Acccgcc | 3.0 |
| AcccgcA | 2.9 | AcccgcG | 2.2 | AcccgcT | 1.9 | AcccgTc | 2.2 |
| AcccgTA | 2.1 | AcccgTG | 2.0 | AcccgTT | 2.0 | AcccgAc | 2.4 |
| AcccgAA | 2.0 | AcccgAG | 1.9 | AcccgAT | 2.1 | AcccgGc | 1.6 |
| AcccgGA | 1.4 | AcccgGG | 1.4 | AcccgGT | 1.4 | AcccCcc | 3.0 |
| AcccCcA | 1.9 | AcccCcG | 2.3 | AcccCcT | 1.9 | AcccCTc | 2.1 |
| AcccCTA | 2.0 | AcccCTG | 2.1 | AcccCTT | 2.0 | AcecCAc | 1.5 |
| AcccCAA | 1.4 | AcccCAG | 1.3 | AcccCAT | 1.3 | AcccCGc | 1.2 |
| AcccCGA | 1.4 | AcccCGG | 1.3 | AcccCGT | 1.2 | AcccAcc | 1.9 |
| AcccAcA | 2.1 | AcccAcG | 1.9 | AcccAcT | 1.9 | AcccATc | 2.2 |
| AcccATA | 1.9 | AcccATG | 1.9 | AcccATT | 2.2 | AcccAAc | 2.0 |
| AcccAAA | 1.9 | AcccAAG | 2.0 | AcccAAT | 2.0 | AcccAGc | 1.3 |
| AcccAGA | 1.1 | AcccAGG | 1.1 | AcccAGT | 1.2 | AcccTcc | 1.1 |
| AcccTcT | 1.0 | AcccTTc | 1.0 | AcccTGc | 1.0 | Gcccgcc | 1.1 |
| GcccgTc | 1.2 | GcccgcA | 1.3 | GcccgGc | 1.3 | GcccCcc | 1.5 |
| GcccTcc | 1.2 | GcccAcc | 1.1 | ggcgggg | 4.5 | agcgggg | 4.9 |
| gacgggg | 4.0 | ttcgggg | 3.9 | ggggggg | 2.0 | ggcggga | 4.0 |
| tgcggga | 2.1 | gacggga | 3.0 | tggggga | 1.9 | ggcgggt | 3.0 |
| wild type (ccccgcc) | | | 5.0 | | | | |
| negative control | | | 1.0 | | | | |

From the above, nucleotide sequence of X1cccX5X6X7 wherein X1 is desired to be c, or t, but may be a; X5 is desired to be g but may be c or a, occasionally t; X6 is desired to be c but may be t or a, occasionally g; X7 is desired to be c, but may be t, a, or g, or nucleotide sequence complementary to these sequence is recognized to have mild low temperature transcription enhancing activity.

### Example 6

It was expected from Example 5 that the nucleotide sequence containing two ccccgcc was mild low temperature transcription enhancer. Accordingly, firefly luciferase cDNA was inserted into multicloning site (between EcoRV and XbaI) of pcDNA3.1(+) vector (Invitrogen) having a CMV promoter and two of
ttccccgccg caggacaaac gggtcgggaa cgcggaagtg ggtcagctcc gcctccaaat cagctgtccc cgcctcctag tcgctag(SEQ ID NO: 3) were incorporated in series 5' to the CMV promoter.

Then 5.0 µg/60 mm dish of this plasmid was transfected into the human cell line U-20S, and the cells were cultured in the presence of G418 (Invitrogen) at 37°C for 3 weeks to have this plasmid integrated into chromosome to become a stably expressing cell clone. The cultured cells were divided into two parts and cultured at 37°C for 2 days before one part was cultured at 32°C and the other part was cultured at 37°C. The medium used was 10% FCS + D-MEM. Cells were recovered by centrifugation at 48 hours after the temperature change, and total cell lysates were prepared. Firefly luciferase activity produced at each temperature was measured using Dual-Luciferase Reporter Assay kit (Promega). The result is shown in Table 6. Firefly luciferase activity is shown as activity per unit weight of extracted protein.

**Table 6**

| Transcription Enhancing Activity of Mild Low Temperature Transcription Enhancer Integrated into Chromosome | | | |
|---|---|---|---|
| Recovered Time | Firefly luciferase activity (per protein weight) | | |
| | 32°C | 37°C | ratio of 32°C/37°C |
| 0 hour | 6500 | 6500 | 1 |
| 48 hour | 26904 | 3116 | 8.63 |

This result shows that even when mild low temperature transcription enhancer was stably integrated into chromosome and different low temperature transcription control elements were connected via other nucleotide sequence, transcription at 32°C was enhanced and the amount of protein produced was increased.

### Example 7

When proteins are produced in mammalian cells, there is a possibility that two protein-expressing genes are cloned into a single expression vector. Thus, to the 5' side of mild low temperature transcription enhancer in the vector used in Example 6 expressing firefly luciferase under control of CMV promoter, another CMV promoter was added in the reverse direction (direction from 3' to 5') to examine its effect on luciferase expression. Like Example 6, the vectors were transfected into the human cell line U-2OS, and the cells were divided into two parts with one part cultured at 32°C and the other at 37°C. It was found that the extra CMV promoter in the reverse orientation at 5' upstream did not decrease the expression, but rather increased the transcription and protein production at 32°C by about 5-100. From this, mild low temperature transcription enhancer can be applied to vectors simultaneously producing two proteins from reverse oriented promoters.

### Example 8

### Gel Shift Assay

Mouse Balb/3T3 fibroblast was cultured in D-MEM supplemented with 10% FCS at 37°C for 24 hours. Part of it was cultured at 32°C and the remainder at 37°C for 8 hours before preparing nuclear protein extracts by conventional method (John David Dignani, Russell M. Lebovitz, and Robert G, Roeder, Accurate transcription initiation by RNA polymerase II in a soluble extract isolated from mammalian nuclei, Nucleic Acids Res., 1983, 11:1475-1489).

Oligonucleotide 5'-ttccccgccgacggatccag-3' (SEQ ID NO:5) was labeled by using T4 polynucleotide kinase and gamma-³⁵-P ATP and purified with Sephadex 50 column (Amersham Bioscience) before it was subjected to annealing with 5'-ctggatccgtcggcggggaa-3' (SEQ ID NO:6) to be a probe with specific activity of 5X105cpm/ng.

0.1 nano gram of this probe and 0.5 micro gram of poly (dI-dC) were mixed with nuclear protein extracts and incubated at 20°C (the composition of this binding buffer was 10 mM Tris (pH7.5), 100 mM NaCl, 2 mM EDTA, 10% glycerol). After 30 minutes, the reaction mixture was subjected to 4% polyacrylamide gel electrophoresis and the gel was dried at 80°C, exposed to X ray film to detect proteins bound to the mild low temperature transcription control elements, i.e. mild low temperature transcription control factors (Figure 3).

### Industrial Availability

The present invention can be widely used for producing proteins.

## Claims

1. A mild low temperature transcription control element consisting of nucleotide sequence of:
X1cccX5X6X7
wherein X1 represents c, t, or a; X5 represents g, c, a, or t; X6 represents c, t, a or g; X7 represents c, a, g or t;
or nucleotide sequence complementary to the sequence.

2. A mild low temperature transcription control element according to Claim 1 which is selected from the group consisting of:
ccccgcc, ccccgca, ccccgcg, ccccgct, ccccgtc, ccccgta, ccccgtg, ccccgtt, ccccgac, ccccgaa, ccccgag, ccccgat, ccccggc, ccccgga, ccccggg, ccccggt, ccccccc, cccccca, ccccccg, cccccct, ccccctc, cccccta, ccccctg, ccccctt, cccccac, cccccaa, cccccag, cccccat, cccccgc, cccccga, cccccgg, cccccgt, ccccacc, ccccaca, ccccacg, ccccact, ccccatc, ccccata, ccccatg, ccccatt, ccccaac, ccccaaa, ccccaag, ccccaat, ccccagc, ccccaga, ccccagg, ccccagt, cccctac, cccctcc, tcccgcc, tcccgca, tcccgcg, tcccgct, tcccgtc, tcccgta, tcccgtg, tcccgtt, tcccgac, tcccgaa, tcccgag, tcccgat, tcccggc, tcccgga, tcccggg, tcccccc, tccccca, tcccccg, tccccct, tcccctc tccccta, tcccctg, tcccctt, tccccac, tccccaa, tccccag, tccccat, tccccgc, tcccacc, tcccaca, tcccacg, tcccact, tcccatc, tcccata, tcccatg, tcccatt, tcccaac, tcccaaa, tcccaag, tcccaat, tcccagc, tcccaga, tccctcc, acccgcc, acccgca, acccgcg, acccgct, acccgtc, acccgta, acccgtg, acccgtt, acccgac, acccgaa, acccgag, acccgat, acccggc, acccgga, acccggg, acccggt, acccccc, accccca, acccccg, accccct, acccctc, accccta, acccctg, acccctt, accccac, accccaa, accccga, acccacc, acccaca, acccacg, acccact, acccatc, acccata, acccatg, acccatt, acccaac, acccaaa, acccaag, acccaat, gcccccc, ggcgggg, agcgggg, gacgggg, ttcgggg, ggggggg, ggcggga, tgcggga, gacggga, tggggga, and ggcgggt.

3. A mild low temperature transcription enhancer in which identical or different, mild low temperature transcription control elements of Claim 1 are connected, provided that mild low temperature transcription control element excludes nucleotide sequence which contain only nucleotide sequence of ccccgcc, ccccgtc, ccccgct or ccccgaa which follow 3' to t; nucleotide sequence of ccccgcc which follow 3' to a; nucleotide sequence of ccccgcc which follow 3' to a; nucleotide sequence of ggcgggg or ggggggg which precede a at 3'terminus as mild low temperature transcription control elements.

4. A mild low temperature transcription enhancer in which identical or different, mild low temperature transcription control elements of Claim 1 are connected via other nucleotide residue, provided that mild low temperature element excludes nucleotide sequence which contain only nucleotide sequence of ccccgcc, ccccgtc, ccccgct or ccccgaa which follow 3' to t; nucleotide sequence of ccccgcc which follow 3' to a; nucleotide sequence of ccccgcc which follow 3' to a; nucleotide sequence of ggcgggg or ggggggg which precede a at 3'terminus as mild low temperature transcription control elements.

5. A mild low temperature transcription enhancer having nucleotide sequence of:
ttccccgccg caggacaaac gggtcgggaa cgcggaagtg ggtcagctcc gcctccaaat cagctgtccc cgcctcctag tcgctag (SEQ ID NO:3)

6. A vector comprising mild low temperature transcription control element of Claim 1 or 2, or mild low temperature transcription enhancer of any of Claims 3-5.

7. A eukaryotic cell tansfected by the vector of Claim 6.

8. A eukaryote tansfected by the vector of Claim 6.

9. A method for assaying mild low temperature transcription control factor or regulatory substance thereof comprising:
incubating double-stranded DNA probe containing mild low temperature transcription control elements with protein solution to be assayed;
measuring mild low temperature transcription control factors by detecting the double-stranded DNA probe bound to them.

10. A method for purifying mild low temperature transcription control factors comprising:
incubating double-stranded DNA probe containing mild low temperature transcription control elements with protein solution;
obtaining mild low temperature transcription control factors specifically bound to mild low temperature transcription control elements; and
isolating mild low temperature transcription control factors.
